(19) Europäisches Patentamt
European Patent Office
Office européen des brevets

(11) Numéro de publication: **0 165 881 B1**

# (12) FASCICULE DE BREVET EUROPEEN

(45) Date de publication du fascicule du brevet: **30.03.88**

(51) Int. Cl.⁴: **C 07 C 45/49**, C 07 C 47/06, C 07 C 47/02, C 07 C 47/228

(21) Numéro de dépôt: **85420083.9**

(22) Date de dépôt: **07.05.85**

## (54) Procédé de préparation d'aldéhydes.

(30) Priorité: **15.05.84 FR 8407697**

(43) Date de publication de la demande: **27.12.85 Bulletin 85/52**

(45) Mention de la délivrance du brevet: **30.03.88 Bulletin 88/13**

(84) Etats contractants désignés: **AT BE CH DE FR GB IT LI LU NL SE**

(56) Documents cités:
**US - A - 4 338 467**

**CHEMICAL ABSTRACTS, vol. 94, no. 7, 16 février 1981, page 543, no. 46969s, Columbus, Ohio, US; & JP - A - 80 31 128 (AJINOMOTO CO. INC.) 15-08-1980**

(73) Titulaire: **RHONE-POULENC CHIMIE, 25, quai Paul Doumer, F-92408 Courbevoie Cédex (FR)**

(72) Inventeur: **Leconte, Philippe, 45, rue Duquesne, F-69006 Lyon (FR)**

(74) Mandataire: **Cazes, Jean-Marie et al, RHONE-POULENC INTERSERVICES Service Brevets Chimie 25, quai Paul Doumer, F-92408 Courbevoie Cédex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

## Description

La présente invention a pour objet un procédé de préparation d'aldéhydes par hydrocarbonylation d'halogénures organiques.

L'hydrocarbonylation des halogénures organiques a donné lieu à de nombreuses recherches en vue de la mise au point d'un procédé industriel de préparation des aldéhydes. Ainsi on a préconisé dans le brevet No. 150 412 (Chemical Abstracts 60 2847c) de procéder à l'hydrocarbonylation des halogénures organiques (par exemple du bromure d'octyle et du chlorure de benzyle) par réaction de ces derniers avec un mélange d'oxyde de carbone et d'hydrogène sous pression, en présence de dicobaltoctacarbonyle dans un mélange acétone/eau. Dans ces conditions les rendements sont souvent modestes et la durée de réaction de l'ordre de 4 heures limite la productivité du procédé malgré le recours à des températures et des pressions élevées et à une quantité relativement importante de catalyseur. Pour diminuer la température de la réaction et sa durée on a proposé dans la demande de brevet français No. 73/46 069, publiée sous le No. 2 211 451, et dans la demande de brevet japonais publiée sous le No. 75/149 610 de préparer le phénylacétaldéhyde et des dérivés de substitution par hydrocarbonylation des halogénures de benzyle en présence d'un catalyseur consistant en un dérivé d'un métal de transition, notamment du dicobaltoctacarbonyle, dans un couple eau/solvant organique non miscible à l'eau en opérant en outre en présence d'une amide tertiaire (diméthyl- ou diéthylformamide ou acétamide) et un d'agent basique servant à neutraliser l'hydracide produit par la réaction (sels de métaux alcalins ou alcalino-terreux d'acides faibles; hydroxydes alcalins; bases organiques). Bien que la présence de l'amide tertiaire permette d'abaisser la température et d'augmenter le rendement en phénylacétaldéhyde celui-ci ne dépasse pas 60% dans le meilleur des cas et les pressions mises en œuvre restent élevées (de l'ordre de 13,7 à 19,6 MPa). Ce procédé présente en outre l'inconvénient de nécessiter l'emploi d'une quantité importante d'amide (1 mole par mole d'halogénure de benzyle) et encore relativement élevée de catalyseur (par exemple 0,26 mole de dicobaltoctacarbonyle par mole d'halogénure de benzyle). Dans la demande de brevet européen No. 0 034 430 on a tenté de résoudre le problème de l'hydrocarbonylation des halogénures d'arylméthyle en arylacétaldéhydes en conduisant la réaction dans un nitrile éventuellement associé à un hydrocarbure en présence d'un dérivé du cobalt et d'une base. La substitution du système nitrile/hydrocarbure au système eau/solvant organique non miscible à l'eau se traduit per l'obtention de bons rendements en arylacétaldéhydes en présence de faibles quantités de catalyseur. On constate néanmoins que la durée de réaction reste de l'ordre de 2 à 3 heures.

En résumé, malgré les travaux effectués depuis la publication du brevet hongrois No. 150 412, l'industrie est encore à la recherche d'un procédé de préparation des aldéhydes par hydrocarbonylation des halogénures organiques qui permette d'atteindre à la fois de bons rendements en aldéhydes et une bonne productivité sans exiger l'emploi de quantité importante de catalyseur d'hydrocarbonylation et le recours à des températures et/ou des pressions élevées. La présente invention a précisément pour objet un procédé d'hydrocarbonylation des halogénures organiques procurant des rendements élevés en aldéhydes et exempts des inconvénients de procédés de l'art antérieur.

Plus spécifiquement la présente invention a pour objet un procédé de préparation d'aldéhydes par réaction d'un halogénure organique avec un mélange gazeux d'hydrogène et d'oxyde de carbone, en presence d'un catalyseur d'hydrocarbonylation et d'un agent de neutralisation des hydracides caractérisé en ce que la réaction d'hydrocarbonylation est conduite dans un acide carboxylique liquide inerte dans les conditions de température et de pression mises en œuvre.

Plus spécifiquement encore la présente invention concerne un procédé de préparation d'aldéhydes de formule générale:

$$R{-}CH_2{-}CHO \quad (I)$$

dans laquelle R représente un atome d'hydrogène, un radical alkyle ou un radical aryle, par hydrocarbonylation à l'aide d'un mélange d'oxyde de carbone et d'hydrogène d'halogénures organiques de formule générale:

$$R{-}CH_2X \quad (II)$$

dans laquelle R a la signification donnée ci-avant et X représente un atome d'halogène pris dans le groupe formé par le chlore, le brome et l'iode, en présence d'un catalyseur d'hydrocarbonylation et d'un agent de neutralisation des hydracides au sein d'un acide carboxylique.

Dans les formules (I) et (II) R représente plus particulièrement un radical alkyle linéaire ou ramifié comportant de 1 à 20 atomes de carbone et de préférence un radical alkyle ayant de 1 à 10 atomes de carbone. Plus spécifiquement R peut représenter un radical méthyle, éthyle, n-propyle, isopropyle, n-butyle, isobutyle, t-butyle, sec-butyle, n-pentyle, méthyl-2 pentyle, éthyl-3 pentyle, n-hexyle, éthyl-2 hexyle, n-octyle. Lorsque R désigne un radical aryle, il représente alors des radicaux phényle ou phényle substitués de formule:

(R')n 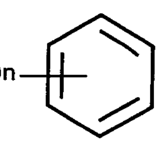 (III)

dans laquelle:

— R' représente un radical alkyle, alkoxy, perhalogénométhyle ou un atome d'halogène,

— n est un nombre entier de 1 à 3.

De préférence R' représente: un radical alkyle linéaire ou ramifié comportant de 1 à 4 atomes de carbone tel que les radicaux méthyle, éthyle, n-propyle, isopropyle, n-butyle, sec-butyle; un radical alkoxy

comportant de 1 à 4 atomes de carbone dans le reste alkyle tels que les radicaux méthoxy, éthoxy, propyloxy; un atome de chlore, de brome ou de fluor; un radical trichlorométhyle ou trifluorométhyle. Parmi les halogénures organiques qui peuvent être hydrocarbonylés par le procédé selon l'invention, on peut citer le chlorure de méthyle, le bromure de méthyle, l'iodure de méthyle, le chlorure d'éthyle, l'iodure d'éthyle, le chlorure de n-propyle, l'iodure de n-propyle, le chlorure de n-butyle, le chlorure et le bromure d'isobutyle, le chlorure et l'iodure de n-pentyle, le chlorure, le bromure et l'iodure de méthyl-2 ou méthyl-3 butyle, le chlorure et l'iodure de benzyle, le chlorure et l'iodure de p-méthylbenzyle, le chlorure et le bromure de p- ou m-chlorobenzyle, le chlorure et l'iodure de p-trifluorométhylbenzyle, le chlorure et l'iodure de m- ou p-méthoxybenzyle.

Comme milieu réactionnel on peut utiliser tout acide carboxylique liquide inerte dans les conditions de la réaction. On a recours de préférence à des acides aliphatiques ou cycloaliphatiques saturés. Pour des raisons pratiques on fait appel de préférence à des acides liquides à température ambiante et à pression normale. Plus particulièrement on fait appel à des acides alcanoïques linéaires ou ramifiés comportant de 1 à 8 atomes de carbone tels que l'acide formique, l'acide acétique, l'acide propionique, l'acide butyrique, l'acide isobutyrique, l'acide méthyl-3 butanoïque, l'acide méthyl-2 butanoïque, l'acide éthyl-2 butanoïque, l'acide diméthyl-2,2 butanoïque, l'acide pentanoïque, l'acide méthyl-2 pentanoïque, l'acide méthyl-5 pentanoïque, l'acide hexanoïque, l'acide éthyl-2 hexanoïque, ou cycloalcanoïque tel que l'acide cyclopentanecarboxylique, les acides méthylcyclopentanecarboxyliques. Pour des raisons de commodité on a recours préférentiellement aux acides alcanoïques comportant de 1 à 4 atomes de carbone. L'acide acétique convient tout particulièrement bien. Bien qu'il soit préférable de conduire la réaction en absence d'eau, celle-ci n'est pas gênante dans la mesure où elle ne représente pas plus de 40% et de préférence 20% du volume de l'acide carboxylique. On peut donc faire appel à un acide anhydre ou à un acide contenant une petite quantité d'eau.

Comme agent de neutralisation de l'hydracide formé au cours de la réaction on peut utiliser tout agent basique approprié. On peut faire appel de préférence:

a) à des amines de formule générale:

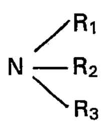

(I)

dans laquelle $R_1$, $R_2$, $R_3$, identiques ou différents, représentent un radical alkyle linéaire ou ramifiée, un radical cycloalkyle, un radical aryle, un radical alkylaryle, un radical arylalkyle, deux au plus des radicaux $R_1$, $R_2$ et $R_3$ pouvant représenter un atome d'hydrogène; dans la formule (I) les restes $R_1$, $R_2$ et $R_3$ représentent de préférence des radicaux alkyles comportant de 1 à 30 atomes de carbone (préférentiellement de 1 à 20); tels que les radicaux méthyle, éthyle, n-propyle, isopropyle, n-butyle, isobutyle, tertiobutyle, n-pentyle, isoamyle, n-hexyle, éthyl-2 butyle, n-heptyle, n-octyle, éthyl-2 hexyle, dodécyle, un radical cyclohexyle ou cyclopentyle, un radical benzyle, phényl-2 éthyle; un radical phényle, toluyle, xylyle. Comme exemple d'amines de formule (I) on peut citer la méthylamine, la diméthylamine, la triméthylamine, l'héthylamine, la di- et la triéthylamine, la diisopropylamine, la triisopropylamine, la diisobutylamine, la di-n-pentylamine, la N,N-diéthylamine, la N,N-diméthylaniline, la N-éthylaniline, la benzylamine, la n-butylcyclohexylamine, la cyclohexylamine, la n-décylamine, la laurylamine. On fait appel de préférence aux amines secondaires et tertiaires.

b) à des bases hétérocycliques azotées comportant un ou deux atomes d'azote intracycliques telles que la pyridine et ses dérivés de substitution comportant 1 à 3 radicaux alkyles ayant de 1 à 4 atomes de carbone, la pipéridine et ses dérivés de substitution comportant 1 à 3 radicaux alkyles inférieurs. Comme base hétérocyclique on peut citer notamment la pyrydine, les α, β et γ-picoline, la lutidine-2,3, la lutidine-2,4, la lutidine-3,5, l'éthyl-2 pyridine, la triméthyl-2,4,6 pyridine, la pipéridine, la n-butyl-1 pipéridine, la diméthyl-1,2 pipéridine, la méthyl-1 pipéridine, l'éthyl-1 pipéridine.

c) à des phosphines de formule générale:

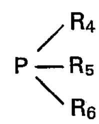

(II)

dans laquelle $R_4$, $R_5$ et $R_6$, identiques ou différents, représentent des radicaux alkyles linéaires ou ramifiés ayant de 1 à 30 atomes de carbone, de préférence 1 à 20; des radicaux cycloalkyle comportant de 5 à 6 atomes de carbone; des radicaux arylalkyle comportant de 1 à 4 atomes de carbone dans le reste alkyle, deux au plus des radicaux $R_4$, $R_5$ ou $R_6$ pouvant représenter un radical aryle, éventuellement substitué par un ou plusieurs radicaux alkyles inférieurs, un radical de formule générale:

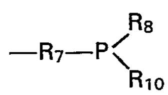

(III)

dans laquelle $R_7$ est un radical alkylène linéaire ou ramifié comportant de 1 à 20 atomes de carbone, de préférence de 2 à 10; $R_8$ et $R_9$, représentent un radical alkyle, un radical cycloalkyle aryle ou arylalkyle tels que ceux définis pour $R_4$ à $R_6$. Comme exemples de radicaux $R_4$, $R_5$, $R_6$ on peut citer les radicaux méthyle, éthyle, n-propyle, isopropyle, n-butyle, isobutyle, tertiobutyle, n-pentyle, isoamyle, n-hexyle, éthyl-2 butyle, n-heptyle, n-octyle, éthyl-2 hexyle, dodécyle, cyclopentyle, cyclohexyle, méthylcyclohexyle, benzyle, phényle, toluyle; $R_7$ peut être un radical méthylène, éthylène, propylène, butylène-1,4, hexaméthylène. Comme exemple de phosphines de

formule (II) on peut citer: la triméthylphosphine, la triéthylphosphine, la tri-n-propylphosphine, la tri-n-butylphosphine, la méthyldiéthylphosphine, l'éthyl-di-n-propylphosphine, la méthyldiisopropylphosphine, la triisopropylphosphine, la phényldiméthylphosphine, le bis(diphénylphosphino)-1,2 éthane.

d) à des sels de métaux alcalins ou alcalino-terreux d'acides carboxyliques de formule générale:

$$R—(—COOM)_n \qquad (IV)$$

dans laquelle:

— R représente un lien valentiel ou un reste hydrocarboné mono- ou polyvalent,

— M représente un métal alcalin tel que Na, K, Li ou un métal alcalino-terreux,

— n est un nombre entier de 1 à 3.

Plus spécifiquement R représente un reste hydrocarboné aliphatique saturé, linéaire ou ramifié, comportant de 1 à 30 atomes de carbone; un reste hydrocarboné cycloaliphatique saturé ayant de 5 à 6 atomes de carbone; un reste aromatique mono- ou polycyclique; un reste arylaliphatique saturé. Plus spécifiquement encore R peut représenter un radical alkyle tel que méthyle, éthyle, n-propyle, isopropyle, n-butyle, sec-butyle, isobutyle, t-butyle, n-pentyle, isoamyle, n-hexyle, éthyl-2 butyle, n-heptyle, n-octyle, éthyl-2 hexyle, undécyle, dodécyle, stéaryle, méthylène, éthylène, triméthylène, tétraméthylène, hexaméthylène, phényle, toluyles, xylyles, benzyle, cyclopentyle, cyclohexyle. Parmi les sels de formule (IV) ou peut citer les acétates de sodium, de potassium, de lithium, de calcium, les propionates, butyrates, pentanoates, hexanoates, éthyl-2 hexanoates, octanoates, décanoates, undécanoates, stéarates, cyclohexanoates, benzoates, toluates, phénylacétates et oxalates de Na, K, Li, Ca, Ba. On peut également utiliser des mélanges de sels alcalins ou alcalino-terreux de mélanges d'acides carboxyliques à forte condensation en carbone comme les acides naphténiques. Parmi ces sels alcalins on choisit de préférence, pour des raisons pratiques, ceux dérivés de l'acide utilisé comme milieu réactionnel.

e) à des carboxylates de phosphonium ou d'ammonium de formule générale:

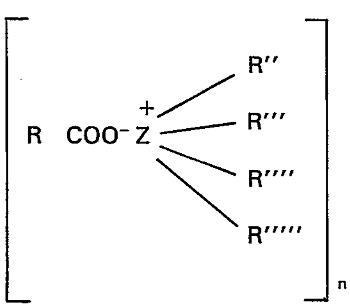

(V)

dans laquelle:

. R et n ont la signification donnée ci-avant pour la formule (IV)

. Z représente un atome d'azote ou de phosphore

. R'', R''', R'''' et R''''' représentent un atome d'hydrogène lorsque Z représente un atome d'azote ou des radicaux hydrocarbonés, identiques ou différents. Plus spécifiquement R'', R''', R'''' et R''''' peuvent représenter des radicaux alkyles linéaires ou ramifiés ayant de 1 à 30 atomes de carbone (de préférence de 1 à 20) tels que les radicaux méthyle, éthyle, n-propyle, isopropyle, n-butyle, isobutyle, tertiobutyle, n-pentyle, isoamyle, n-hexyle, éthyl-2 butyle, n-heptyle, n-octyle, éthyl-2 hexyle, dodécyle, cyclopentyle, cyclohexyle, méthylcyclohexyle; des radicaux cycloalkyles ayant de 5 à 6 atomes de carbone cyclique tels que les radicaux cyclopentyle, cyclohexyle, méthylcyclohexyle; des radicaux aryles éventuellement substitués par un ou plusieurs radicaux alkyles inférieurs (ayant de 1 à 4 atomes de carbone) tels que les radicaux phényle, toluyles, ou xylyles; des radicaux arylalkyles comportant de 1 à 4 atomes de carbone dans le reste alkyle tels que les radicaux benzyle, phényl-2 éthyle. Comme exemple représentatif de ces sels on peut citer: l'acétate, le propionate, l'éthyl-2 hexanoate, le stéarate et le benzoate d'ammonium; les acétates de tétraméthylphosphonium, de tétraéthylphosphonium, du méthyltrybutylphosphonium, l'acétate de tétraéthylammonium, de tétrabutylammonium, de méthyltributylammonium, le propionate de tétraphénylphosphonium.

Lorsqu'on fait appel à des carboxylates alcalins ou alcalino-terreux ou d'ammonium, on peut les charger à l'état préformé ou bien les former «in situ» en introduisant la base correspondante dans l'acide carboxylique utilisé comme milieu réactionnel. En raison de la nature du milieu réactionnel on fait appel de préférence à des carboxylates et en particulier à des sels alcalins ou alcalino-terreux ou d'ammonium dérivés de l'acide carboxylique utilisé ou de tout autre acide carboxylique; ainsi les sels d'acides carboxyliques qui ne sont pas liquides dans les conditions de la réaction tels que les octanoates ou benzoates peuvent être employés.

La quantité d'agent de neutralisation des hydracides exprimée en équivalent molaire de base par mole d'halogénure organique peut varier dans de larges limites. De préférence cette quantité est au moins voisine de la quantité stoechiométrique c'est-à-dire d'un équivalent molaire de base par mole d'halogénure organique. Bien qu'il n'y ait pas de limite supérieure critique de cette quantité, le recours à un rapport équivalent molaire de base/mole d'halogénure organique supérieur à 3 ne se traduit par aucun avantage particulier. A titre préférentiel la quantité d'agent de neutralisation représente de 0,9 à 2,5 équivalents molaires par mole d'halogénure organique et de préférence de 1 à 2 équivalents molaires.

Comme catalyseurs on peut faire appel aux dérivés des métaux de transition habituellement utilisés dans les réactions d'hydrocarbonylation tels que ceux cités dans la demande de brevet français 73/46.069 à laquelle il a été fait référence ci-avant. On met en œuvre de préférence des dérivés carbonylés des métaux du groupe VIII de la classification périodique des éléments (cf. Handbook of Chemistry and Physics 53ème édition, édité par «The Chemical Rubber Company» 1972-73) et notamment du fer, du cobalt et du nickel. On fait appel préférentiellement au dicobaltoctacarbonyle, au tétracobaltdodécacarbonyle et aux sels de l'hydrure de

tétracarbonylcobaltate(-1) [$HCo(CO)_4$] et notamment ceux dérivés des métaux alcalins ou alcalino-terreux, d'ammonium, de phosphonium tels que les tétracarbonylcobaltates(-1) de sodium, de potassium, de lithium, de calcium, d'ammonium, de tétraéthylammonium, de méthyltriphénylphosphonium. On peut également utiliser des mélanges de ces cobaltcarbonyles.

La quantité de catalyseur exprimée en atome-gramme de métal par mole d'halogénure organique peut varier dans de larges limites. Ainsi, et cela constitue un des avantages du procédé selon l'invention la quantité de catalyseur d'hydrocarbonylation peut représenter seulement 0,001 (de préférence 0,01) atome-gramme de métal par mole d'halogénure organique. Bien qu'il n'y ait pas de limite supérieure critique de la quantité de catalyseur, il n'est pas nécessaire de dépasser 0,5 atome-gramme de cobalt par mole d'halogénure organique; une quantité inférieure ou égale à 0,35 atome-gramme par mole d'halogénure organique est en général suffisante. Naturellement on pourrait avoir recours à des quantités de catalyseur sortant des limites définies ci-avant sans pour autant sortir du cadre de la présente invention.

La température à laquelle on conduit la réaction dépend dans une large mesure du substrat soumis à l'hydrocarbonylation. D'une manière générale cette température peut être comprise dans une échelle de 20 à 150°C; cependant il est préférable dans le cas de l'hydrocarbonylation des halogénures de benzyle de ne pas dépasser une température de 100°C. En définitive la température est de préférence comprise entre 50 et 120°C.

La pression totale d'oxyde de carbone et d'hydrogène mise en œuvre pour assurer le déroulement de la réaction peut varier dans de larges limites. Ainsi elle peut être d'au moins 1 MPa. Bien qu'elle puisse atteindre les valeurs habituellement utilisés dans ce type de réaction, il n'est pas nécessaire de recourir à des pressions supérieures à 12 MPa et de préférence à 11 MPa. Ceci constitue un autre avantage du procédé selon l'invention. En général une pression totale comprise dans une échelle de 2 à 10 MPa convient bien.

Les quantités d'oxyde de carbone et d'hydrogène mises en œuvre doivent être suffisantes pour assurer la transformation complète de l'halogènure organique. Au delà de ces quantités il n'y a pas de limites supérieures critiques.

La composition du mélange gazeux oxyde de carbone/hydrogène n'est pas critique et peut varier dans de larges limites. On peut utiliser indifféremment un excès de l'un ou l'autre des constituants du mélange. Ainsi le rapport molaire $CO/H_2$ peut varier de 1/10 à 10 et de préférence de 1/4 à 4.

Sur un plan pratique le procédé selon l'invention ne présente pas de difficultés particulières et peut aisément être mis en œuvre industriellement, en particulier de façon continue. En règle générale il suffit de charger l'acide carboxylique, l'halogénure organique, le catalyseur et l'agent de neutralisation des hydracides dans un appareil résistant à la pression, puis de purger ce dernier par un courant de $CO/H_2$, d'établir une pression adéquate de $CO/H_2$ et de porter le contenu de l'appareil à la température choisie. Lorsque l'absorption de $CO/H_2$ cesse, la masse réactionnelle est refroidie à température ambiante et l'aldéhyde formé est récupéré par mise en œuvre des techniques usuelles, par exemple par distillation. L'acide carboxylique contenant le catalyseur peut être recyclé à une nouvelle opération d'hydrocarbonylation. Dans le cas de l'hydrocarbonylation des halogénures de benzyle il est préférable de charger ce dernier dans l'appareil au moyen d'une pression d'oxyde de carbone après établissement de la température et de la pression adéquate. L'introduction de l'halogénure de benzyle peut être réalisée en une ou plusieurs fois et de manière continue ou discontinue.

La durée de la réaction dépend des conditions choisies.

Les exemples suivants illustrent l'invention et montrent comment elle peut être mise en pratique.

*Exemple 1*

Dans un autoclave en tantale agité par secousses de 120 ml on charge:

| | | | |
|---|---|---|---|
| — | 50 | ml d'acide acétique | |
| — | 2,02 | g de chlorure de méthyle | (40 mMol) |
| — | 4,1 | g d'acétate de sodium | (50 mMol) |
| — | 0,646 | g de dicobaltoctacarbonyle | (3,8 mAt-g) |

On purge l'autoclave par un courant gazeux d'un mélange de $CO/H_2$ (1 mole/2 moles), on le ferme et établit une pression de $CO/H_2$ de 7,9 MPa. On porte ensuite son contenu à 80°C à l'aide d'un four annulaire. On maintient la pression constante et égale à 9,5 MPa par alimentation d'un mélange équimoléculaire de $CO/H_2$ en continu. Après 2 h 10 mn dans ces conditions, l'absorption de $CO/H_2$ cesse. La masse réactionnelle est refroidie à 20°C et l'autoclave est dégazé. Le mélange réactionnel est analysé par chromatographie en phase gazeuse (CPG). On constate que la totalité du chlorure de méthyle a été transformée. Il s'est formé 1,7 g d'acétaldéhyde ce qui représente un rendement par rapport au chlorure de méthyle chargé (RR) de 97%.

*Exemples 2 à 3*

On a opéré comme à l'exemple 1 après avoir remplacé le chlorure de méthyle par l'iodure d'éthyle (6,24 g) et l'iodure de n-butyle (7,36 g) toutes choses égales par ailleurs.

On a obtenu les résultats consignés dans le tableau suivant:

| Exemples | halogénure organique | TT % (1) | Durée en mn | RR % en aldéhyde |
|---|---|---|---|---|
| 2 | Iodure d'éthyle | 100 | 80 | 87% propanol |
| 3 | Iodure de n-butyle | 100 | 110 | 86% pentanal |

(1) taux de transformation.

*Exemple 4*

On a répété l'exemple 1 après avoir remplacé le chlorure de méthyle par l'iodure de méthyle (5,68 g) toutes choses égales par ailleurs exception faite de ce que la pression est maintenue à 9,5 MPa par alimentation continue d'un mélange $CO/H_2$ en 1/2 en moles. La durée de l'absorption est de 80 minutes dans ces conditions, le taux de transformation de l'iodure de méthyle de 100% et le RR en acétaldéhyde de 95%.

*Exemples 5 à 7*

On a opéré comme à l'exemple 4 mais en faisant varier la quantité de dicobaltoctacarbonyle et après avoir porté la quantité d'acétate de sodium à 8,2 g (100 mMol). On a obtenu les résultats consignés dans le tableau suivant:

| Exemples | $Co_2(CO)_8$ | Durée mn | TT % | RR en acétaldéhyde % |
|---|---|---|---|---|
| 5 | 321 mg<br>1,88 mAt.g | 100 | 100 | 95 |
| 6 | 637 mg<br>3,7 mAt.g | 40 | 100 | 95 |
| 7 | 1266 mg<br>7,4 mAt.g | 25 | 100 | 95 |

*Exemple 8*

Dans un autoclave en acier inoxydable de 250 $cm^3$ agité par un dispositif à secousses et chauffé par un four annulaire on charge:

- acide acétique 80 ml
- acétate de sodium 6,31 g (77 mMol)
- dicobaltoctacarbonyle 2,07 g (12,1 mAt-g).

Après purge de l'autoclave on introduit un mélange équimoléculaire de $CO/H_2$ jusqu'à une pression de 5,2 MPa puis on porte la température du contenu de l'autoclave à 80°C. Dans un deuxième autoclave de 125 ml on charge 10,12 g de chlorure de benzyle (80 mMol) en solution dans 20 ml d'acide acétique et 14 MPa d'hydrogène. Lorsque la température atteint 80°C dans le premier autoclave on le relie au second da façon à charger la solution acétique de chlorure de benzyle. La pression est maintenue à 9,5 MPa par alimentation en continu d'un mélange équimoléculaire de $CO/H_2$. Après 30 mn l'absorption cesse. Le mélange réactionnel est traité et analysé comme à l'exemple 1. Le taux de transformation du chlorure de benzyle est de 96% et le rendement en phénylacétaldéhyde par rapport au chlorure de benzyle chargé s'élève à 87%.

*Exemple 9*

On a reproduit l'exemple 8 après avoir porté la quantité de $Co_2(CO)_8$ de 2,07 g à 2,16 g (12,6 mAt-g) et la température de 80 à 100°C. La durée de réaction est de 20 mn, le taux de transformation du chlorure de benzyle de 65% et le RR de 45% ce qui correspond à un rendement de 69,2% en phénylacetaldéhyde par rapport au chlorure de benzyle transformé (RT).

*Exemple 10*

Dans un autoclave en acier inoxidable de 80 ml équipé comme décrit ci-avant on charge:

- acide acétique 32 ml
- triéthylamine 3,05 g (30 mMol)
- $Co_2(CO)_8$ 1,37 g (8 mAt-g)
- chlorure de benzyle 3,3 g (26 mMol) dans un tube en verre de hauteur supérieure au niveau du liquide présent dans l'autoclave.

Après purge de ce dernier on introduit 7,9 MPa d'un mélange équimoléculaire de $CO/H_2$. La température est ensuite portée à 80°C l'agitation est mise en marche et la pression maintenue à 9,5 MPa comme précédemment. Après 40 mn l'absorption cesse. Le mélange réactionnel est traité et analysé comme aux exemples précédents. Le taux de transformation du chlorure de benzyle s'élève à 100% et le RR en phénylacétaldéhyde à 80%.

*Exemple 11*

On a répété l'exemple 10 après avoir remplacé la triéthylamine par 3,18 g d'acétate de calcium (20 mMol) et ramené la quantité de $Co_2(CO)_8$ à 1,12 g (6,6 mAt-g). Le taux de transformation du chlorure de benzyle s'est élevé à 60% pour une durée de 30 mn et le RR à 65% (soit un RT de 89%).

*Exemples 12 à 16*

Dans l'autoclave décrit à l'exemple 10 et en suivant le même mode opératoire on a conduit cinq essais dont les conditions et les résultats sont consignés dans le tableau suivant (dans tous les cas on a chargé 40 mMol de chlorure de benzyle et la température a été de 80°C).

| Exemples | Milieu (en ml) | Acétate de sodium en mMol | $Co_2(CO)_8$ en mAt-g de Co | Pression en MPa | Durée en mn | TT % | RR % | RT (2) % |
|---|---|---|---|---|---|---|---|---|
| 12 | $CH_3COOH$ (45) | 50 | 3,27 | 9,5 | 70 | 81 | 71 | 87,6 |
| 13 | $CH_3COOH$ (30)<br>eau (20) | 42 | 3,8 | 9,5 | 140 | 62 | 34 | 54,8 |
| 14 | $CH_3COOH$ (40)<br>eau (10) | 42 | 3,2 | 9,5 | 60 | 67 | 57 | 85 |
| 15 | $CH_3COOH$ (45) | 40 | 3,5 | 3 | 40 | 54 | 34 | 62,9 |
| 16 (1) | $CH_3COOH$ (45) | 40 | 3,5 | 6 | 100 | 65 | 54 | 83 |

(1) la pression de 6 MPa a été établie au moyen d'un mélange $CO/H_2$ 2/1 en mole puis maintenue au moyen d'un mélange équimoléculaire de $CO/H_2$.
(2) Rendement en phénylacétaldéhyde par rapport au chlorure de benzyle transformé.

*Exemple 17*

On a opéré comme à l'exemple 1 dans les conditions suivantes:

- chlorure de benzyle 3,26 g (25,8 mMol)
- acide acétique 32 ml
- diisopropylamine 3,04 g (30,1 mMol)
- $Co_2(CO)_8$ 1,34 g (7,8 mAt-g)
- température 80°C
- pression totale 9,5 MPa
- $CO/H_2$ 1

L'absorption cesse après 20 minutes dans ces conditions. La totalité du chlorure de benzyle a été transformée et le dosage chromatographique fait apparaître un rendement en phénylacétaldéhyde de 85%.

**Revendications**

1. Procédé de préparation d'aldéhydes par réaction d'un halogénure organique avec un mélange gazeux d'hydrogène et d'oxyde de carbone, en présence d'un catalyseur d'hydrocarbonylation et d'un agent de neutralisation des hydracides caractérisé en ce que la réaction d'hydrocarbonylation est conduite dans un acide carboxylique liquide inerte dans les conditions de température et de pression mises en œuvre.

2. Procédé selon la revendication 1, caractérisé en ce que l'on utilise comme acides carboxyliques des acides alcanoïques ou cycloalcanecarboxyliques.

3. Procédé selon la revendication 2, caractérisé en ce que l'on utilise des acides alcanoïques linéaires ou ramifiés comportant de 1 à 8 atomes de carbone.

4. Procédé selon l'une quelconque des revendications 1 à 3, caractérisé en ce que l'on utilise l'acide acétique comme milieu d'hydrocarbonylation.

**Claims**

1. Process for the preparation of aldehydes by reaction of an organic halide with a gaseous mixture of hydrogen and carbon monoxide, in the presence of a hydrocarbonylation catalyst and an agent for neutralizing the hydrogen acids, characterized in that the hydrocarbonylation reaction is carried out in a liquid carboxylic acid which is inert under the temperature and pressure conditions employed.

2. Process according to Claim 1, characterized in that alkanoic or cycloalkanecarboxylic acids are used as carboxylic acids.

3. Process according to Claim 2, characterized in that straight-chain or branched alkanoic acids containing from 1 to 8 carbon atoms are used.

4. Process according to any one of Claims 1 to 3, characterized in that acetic acid is used as a hydrocarbonylation medium.

**Patentansprüche**

1. Verfahren zur Herstellung von Aldehyden durch Reaktion eines organischen Halogenids mit einem gasförmigen Gemisch von Wasserstoff und Kohlenmonoxid in Gegenwart eines Hydrocarbonylierungskatalysators und eines Neutralisationsagens für Wasserstoffsäuren, dadurch gekennzeichnet, dass die Hydrocarbonylierungsreaktion in einer flüssigen Carbonsäure durchgeführt wird, die unter den angewandten Temperatur- und Druckbedingungen inert ist.

2. Verfahren nach Anspruch 1, dadurch gekennzeichnet, dass man als Carbonsäuren Alkancarbonsäuren oder Cycloalkancarbonsäuren verwendet.

3. Verfahren nach Anspruch 2, dadurch gekennzeichnet, dass man lineare oder verzweigte Alkancarbonsäuren mit 1 bis 8 Kohlenstoffatomen verwendet.

4. Verfahren nach einem der Ansprüche 1 bis 3, dadurch gekennzeichnet, dass man als Hydroxycarbonylierungmilieu Essigsäure verwendet.